# EUROPÄISCHE PATENTANMELDUNG

(11) **EP 1 344 540 A1**
(43) Veröffentlichungstag der Anmeldung: **17.09.2003**
(21) Anmeldenummer: 03405184.7
(22) Anmeldetag: 17.03.2003
(51) Int. Cl.: A61M 1/02, G06F 19/00

(54) **Vorrichtung zur Überwachung von Blutentnahmen**

(30) Priorität: 15.03.2002 CH 4512002
(71) Anmelder: Möller Feinmechanik GmbH & Co. KG, 36043 Fulda (DE)
(72) Erfinder: Heil, Markus, 36039 Fulda (DE); Schröter, Werner, 36137 Grossenlüder (DE)
(74) Vertreter: Walder, Martin B.

(57) **Zusammenfassung**

Blutwaagen (11.1 bis 11.6) stehen mit getrennt von den Blutwaagen vorliegenden Eingabe/Überwachungsgeräten derart in Verbindung, dass die von den Eingabegeräten eingegebenen Bedienungsbefehle einer ausgewählten Blutwaage (11) übermittelt werden, wo sie umgesetzt werden. Bedienungsbefehle sind Befehle, mit denen z.B.
- die Blutwaage auf Null gestellt wird,
- die maximale Blutmenge oder die maximale Entnahmedauer eingestellt wird,
- ein Bewegungsmuster zum Mischen des Blutes mit dem Antikoagulansmittel eingegeben wird,
- festgelegt wird, bei welcher Blutmenge oder Entnahmenzeit, oder bei welcher minimal einfliessenden Blutmenge pro Zeiteinheit die Blutwaage eine Meldung absenden soll,
- ein Alarm-Muster eingegeben wird.
Abgesehen von solchen Bedienungsbefehlen können über ein Eingabe/Überwachungsgerät in bekannter Weise auch medizinische Daten wie Patientendaten, Blutdaten etc. eingegeben werden. Das Eingabe/Überwachungsgerät ist vorteilhaft ein kabellos mit der Blutwaage verbundenes Handgeräten (23.1, 23.2). Die von einer Blutwaage (11) gemessenen Messwerte können auf das Handgerät (23) übertragen werden, wo sie abgelesen werden können. Die Zuordnung von Blutwaage (11) zu Handgerät (23) ist wählbar. Ebenso ist die Zuordnung der Art der Meldungen der Blutwaage (11) an die Handgeräte (23) wählbar.

## Beschreibung

Die Erfindung betrifft eine Vorrichtung zur Überwachung von Blutentnahmen nach dem Oberbegriff des Anspruchs 1.

Bei Blutentnahmen ist ein Gefälle zwischen der Blutentnahmestelle am Blutspender und dem Blutbeutel erforderlich. Dies führt dazu, dass die Blutwaage in der Regel wenige Zentimeter über dem Fussboden angeordnet ist, während der Blutspender auf einer Liege auf einer Höhe liegt, die für das Anstechen einer Vene mit einer Nadel durch eine sitzende Person geeignet ist. Der Blutbeutel liegt zur Kontrolle des Fortschrittes und zur Durchmischung des in den Beutel fliessenden Blutes mit dem darin vorliegenden Antikoagulationsmittels auf einer Blutwaage. Blutwaagen zeigen bei einer Blutentnahme zu überwachende Parameter wie z.B. Gewicht der eingeflossenen Blutmenge, einfliessende Blutmenge pro Zeiteinheit, Dauer der Blutentnahme etc. an. Eine die Blutentnahme überwachende Person muss wiederholt die Blutwaage aufsuchen und sich bei dieser bücken, um sie abzulesen und zu bedienen. Sie wird beispielsweise die Blutwaage nach Auflegen des Beutel auf Null stellen, eine Zielmenge eingeben, deren Erreichen die Blutwaage melden soll, oder eine maximale Entnahmezeit eingeben, deren Ablauf die Blutwaage melden soll. Bei manchen Blutwaagen kann auch ein Bewegungsmuster zum Mischen des einlaufenden Blutes mit dem Antikoagulationsmittel im Blutbeutel eingegeben werden. Weiter sind an den Blutwaagen oft auch Strichcode-Leser angeordnet, mit denen eine Strichcode-Information auf dem Beutel, z.B. die Beutelnummer, oder Patientendaten eingelesen werden können. Diese und weitere Verrichtungen an der Blutwaage müssen praktisch in der Hocke ausgeführt werden. Das Ablesen des Beutelgewichtes ist lediglich auf der Blutwaage, allenfalls noch an einem zentralen Arbeitsplatz, an dem ein mit den Blutwaagen der Blutentnahmestelle verbundener Computer steht, möglich.

Aus der DE-U-296 11 131 ist ein Blutentnahmegerät bekannt, bei dem eine Übertragungseinrichtung zum drahtlosen Übertragen von Daten mit einer externen Zentraleinheit vorgesehen ist, wobei mehrere derartige Blutentnahmegeräte und die externe Zentraleinheit zu einem System gekoppelt sein können. Diese Druckschrift offenbart eine bidirektionale Kommunikation, insofern die Zentraleinheit die verschiedenen Blutentnahmegeräte zyklisch auffordert, die ermittelten Daten zu übertragen.

Aus der EP-A-1 156 441 ist eine bidirektionale Kommunikation mittels jeweils einer Sende/Empfangseinreichtung an einer Datenverarbeitungseinrichtung und einer medizinische Daten liefernden Einrichtung bekannt. Die Sende/Empfangseinrichtungen sind entsprechend des Types der medizinische Daten liefernden Einrichtung konfiguriert, so dass gleichartige Sende/Empfangseinrichtungen bei unterschiedlichen Einrichtungen verwendet werden können. Die Übermittlung zwischen den Sende/Empfangseinrichtungen erfolgt drahtlos.

Aus der US-A- 4 605 080 ist eine Wägeeinrichtung bekannt, die ein Spracherkennungs-Steuersystem aufweist, mit dem die Wägeeinrichtung gesteuert werden kann und mit der weitere Vorgänge eingeleitet und durchgeführt werden können. Diese bekannte Wägeeinrichtung ist ausschliesslich zur Verwendung in einem automatisierten Ablauf zum fortlaufenden Abwiegen und Etikettieren von Massengütern vorgesehen.

Aus der EP-1049 494 ist die Verwendung einer Spracheingabeeinrichtung in einem Blutentnahmegerät bekannt. Das für die Spracheingabe benötigte Mikrofon kann getrennt von dem Blutentnahmegerät an einer Tastatur oder einem Strichcode-Leser angeordnet sein. Die Spracheingabeeinrichtung, die Tastatur und der Strichcode-Leser werden verwendet, um diejenigen Daten zu erfassen, die in für ein jeweiliges Land gültigen Richtlinien zur Blutentnahme festgelegt sind. Die Verbindung von der Tastatur oder dem Strichcode-Leser und dem Mikrofon zum Blutentnahmegerät erfolgt über ein Kabel. Die Übermittlung der Daten vom Blutentnahmegerät zu einer externen Verarbeitungseinheit erfolgt über Kabel oder kabellos. Für die Bedienung des Blutentnahmegeräts können gemäss dieser Druckschrift Bedienelemente am Blutentnahmegerät vorgesehen sein. Mit diesen Bedienelementen kann beispielsweise die zu entnehmende Blutmenge voreingestellt werden.

Es ist deshalb die Aufgabe der Erfindung, die Bedienung einer eine Blutentnahme überwachenden Blutwaage zu erleichtern. Mit einer vorteilhaften Ausführungsform soll die Überwachung einer Blutentnahme erleichtert werden.

Erfindungsgemäss wird dies bei einer eingangs erwähnten Vorrichtung durch die kennzeichnenden Merkmale des Patentanspruchs 1 erreicht. Durch eine Empfangseinrichtung zum Empfangen von elektronisch übermittelten Bedienungsbefehlen und einen Steuerkreislauf zum Ausführen der mit der Empfangseinrichtung empfangenen Bedienungsbefehle ist ermöglicht, dass die Blutwaage von einem zentralen Computerarbeitsplatz oder einem anderen Eingabegerät, z.B. einen Handgerät, aus bedient werden kann. Unter Bedienungsbefehlen werden Befehle verstanden, mit denen die Waage auf Null gestellt wird, die maximale Blutmenge oder die maximale Entnahmedauer eingestellt wird. Es kann ferner ein Bewegungsmuster zum Mischen des Blutes mit dem Antikoagulansmittel eingegeben werden. Es kann beispielsweise eingegeben werden, bei welcher Blutmenge oder Entnahmenzeit, oder bei welcher minimal einfliessenden Blutmenge pro Zeiteinheit die Blutwaage eine Meldung absenden soll. Ferner können über Bedienungsbefehle z.B. auch verschiedene Alarm-Muster eingegeben werden. Abgesehen von solchen Bedienungsbefehlen können über ein Eingabe/Überwachungsgerät in bekannter weise auch medizinische Daten wie Patientendaten, Blutdaten etc. eingegeben werden.

Es ist daher neben einer Blutwaage ein von der Blutwaage getrenntes Eingabe- oder Überwachungsgerät vorhanden. Zwischen Blutwaage und Eingabe/Überwachungsgerät besteht eine drahtgebundene oder kabellose elektronische Verbindung, über welche Informationen ausgetauscht werden können. Solche Informationen sind insbesondere Bedienungsbefehle. Falls eine bidirektionale Kommunikation vorhanden ist, können vorteilhaft auch Messwerte übertragen werden. Ein solches Eingabe/Überwachungsgerät kann auf einer bedienfreundlichen Höhe angeordnet sein oder vorteilhaft gar als mobiles Handgerät ausgelegt sein.

Eine zweckmässige Vorrichtung zur Überwachung einer Blutentnahme weist daher folgende Bestandteile auf:
1. Eine Blutwaage mit
   - einer Beutelschale zum Auflegen und Wägen eines Blutbeutels,
   - einer Absendeeinrichtung zum Senden des Messwertes,
   - einer Empfangseinrichtung, mit der Bedienungsbefehle empfangen werden können,
   - und einem Steuerkreislauf zum Ausführen der mit der Empfangseinrichtung empfangenen Bedienungsbefehle.
2. Ein von der Blutwaage getrenntes Überwachungsgerät mit
   - einer Empfangseinrichtung zum Empfangen eines Messwertes und
   - einer Anzeige zum Anzeigen des Messwertes,
   - einem Eingabefeld zum Eingeben von Bedienungsbefehlen,
   - und einer Absendeeinrichtung, mit der die Bedienungsbefehle an die Blutwaage gesendet werden können.
Diese Merkale gewährleisten, dass die Blutwaage mit dem Überwachungsgerät bedienbar ist und die von der Blutwaage gemessenen Werte auf dem Überwachungsgerät ablesbar sind. Die Verbindung zwischen Blutwaage und Überwachungsgerät kann dabei über Kabel oder kabellos erfolgen. Ebenso kann die Verbindung zwischen einer Blutwaage und einer zentralen Datenverarbeitung über Kabel oder kabellos erfolgen.

Vorteilhaft erfolgt der Informationsaustausch zwischen Blutwaage und Eingabe/Überwachugnsgerät kabellos. Dies ermöglicht eine Veränderung der räumlichen Position des Überwachungsgeräts relativ zur Blutwaage und umgekehrt ohne Installationsaufwand.

Dank der kabellosen Übertragung ist auch ermöglicht, dass das Eingabe/Überwachungsgerät ein mobiles Handgerät ist, das von einer eine Blutentnahme überwachenden Person mitgeführt werden kann. Die Abmessungen eines solchen Handgeräts sind zweckmässigerweise so klein, dass es in eine Kitteltasche passt.

Vorteilhaft sind mehrere Blutwaagen vorhanden und kann das Überwachungsgerät jede der vorhanden Blutwaagen überwachen. Dies erlaubt eine Reduktion der Anzeigen von der Anzahl Blutwaagen auf die Anzahl Überwachungsgeräte oder zumindest eine Verkleinerung der Anzeigen bei den Blutwaagen ohne Komforteinbusse. Sind zudem mehrere Überwachungsgeräte vorhanden, wobei wenigstens ein mobiles Handgerät kabellos mit einer Blutwaage verbunden ist, so sind die Blutwaagen von jedem Überwachungsgerät aus bedienbar. Die Zuordnung der Bedienbarkeit kann vorteilhaft durch eine entsprechende Programmierung der Blutwaage und/oder des Überwachungsgeräts ausgewählt werden, damit die Zuständigkeiten bei mehreren überwachenden Personen eindeutig sind.

Zur Verringerung der Anzahl notwendiger Lesegeräte, z.B. Strichcode-Leser, und zur Komfortsteigerung ist vorteilhaft ein Lesegerät am Überwachungsgerät angeschlossen. Bei Handgeräten ist das Lesegerät vorteilhaft im Handgerät integriert.

Anstelle eines Strichcodelesers ist auch ein anderes berührungslos arbeitendes Lesegerät denkbar. Vorteilhaft ist ein Lese/Schreibgerät am Überwachungsgerät vorgesehen, das beispielsweise mit einem Transponder-Chip oder "Bluetooth"-Chip zusammenarbeitet. Entsprechend sind auf den Blutbeuteln und auf Eingabelisten für Standard-Befehle Transponder oder "Bluetooth"-Chips angeordnet.

Vorteilhaft ist im Überwachungsgerät ein Alarmgeber integriert, der ein akustisches, visuelles und/oder taktiles Signal gibt, wenn eine Blutwaage eine alarmierende Information oder ein elektronisches Alarmsignal sendet.

Das System von Blutwaage und Überwachungsgerät ist vorteilhaft ergänzt mit jeweils einem beschreibbaren und auslesbaren elektronischen Speicher an den Blutbeuteln. Dieser Speicher kann medizinische Daten speichern, die somit unlösbar mit dem Spenderblut enthaltenden Blutbeutel verbunden sind. Dadurch können Verwechslungen praktisch ausgeschlossen werden. Der Speicher kann zudem Transport und Lagerdaten speichern.

Vorteilhaft wird bei jeder Aktion mit dem Blutbeutel der Speicher wenn möglich abgefragt und dessen Inhalt mit dem Inhalt der zentralen Datenbank verglichen. Vorteilhaft wird bei jeder Aktion der Speicherinhalt zumindest ergänzt. Ergänzend sind beispielweise in den Speicher die Handlung und der Zeitpunkt einzuschreiben, an welchem die Handlung stattfand. Lagertemperaturen können im Speicher ebenfalls vermerkt werden. Diese können aber auch lediglich in einem Lagerjournal geführt werden, so dass durch Verbinden der Information auf dem Speicher mit der Information des Lagerjournals ein lückenloser Temperaturverlauf für den Blutbeutel erstellbar ist.

### Kurzbeschreibung der Figuren:

In den Figuren sind Ausführungsbeispiele von erfindungsgemässen Vorrichtungen schematisch dargestellt. Es zeigt:
- Fig. 1: eine Blutentnahme mit einem Blutbeutel auf einer Blutwaage und einem mobilen Handgerät zur Bedienung der Blutwaage,
- Fig. 2: eine Blutwaage und sowohl ein festes wie auch ein mobiles Überwachungsgerät,
- Fig. 3: einem eine Reihe von sechs Blutwaagen mit zwei mobilen Handgeräten und zentralen Computerarbeitsplatz,
- Fig. 4: eine schematische Darstellung eines Blutspendezentrums,
- Fig. 5: einen Blutbeutel mit einem in einer Etikette integrierten Speicher mit Transponderschlaufe.

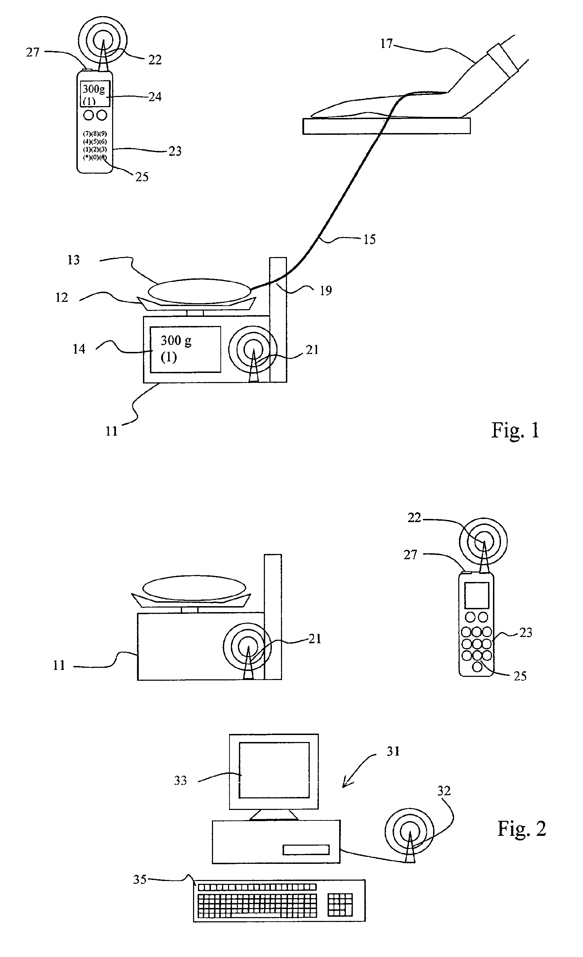

Figur 1 zeigt eine Blutwaage 11, auf deren Blutwaagschale 12 ein Blutbeutel 13 liegt. Über einen Schlauch 15 fliesst Blut aus einem Arm 17 eines Blutspenders in den Blutbeutel 13. Damit das Blut ungepumpt fliesst, ist zwischen dem Arm 17 und dem Blutbeutel 13 ein Niveauunterschied geschaffen. Damit einfliessendes Blut nicht gerinnt, liegt im Blutbeutel eine Vorgabe von Antikoagulationsmittel vor und wird Blut und Antikoagulationsmittel mittels Mischbewegungen der Blutwaagschale 12 miteinander vermischt. Die Menge an Blut im Blutbeutel 13 nimmt während einer Blutentnahme laufend zu. Das jeweilige Gewicht des Blutes wird mit der Blutwaage gewogen. Im Beispiel zeigt die Anzeige 14 der Blutwaage 11 300 Gramm an. Wird eine vorgegebene Blutmenge, z.B. 400 Gramm, erreicht, so stellt die Blutwaage selbsttätig die Blutzufuhr ab, indem sie den Schlauch 15 mit einer Klemmvorrichtung 19 abklemmt. Dazu ist der Schlauch 15 durch die Klemmvorrichtung 19 hindurchgeführt.

Die gewogene Blutmenge und das Erreichen der vorgegebenen Blutmenge wird auch mit einem Sender 21 (dargestellt durch eine Antenne) an ein Handgerät 23 übermittelt. Dieses ist dazu mit einem Empfänger 22 ausgerüstet (dargestellt durch eine Antenne). Auf der Anzeige 24 von diesem sind dieselben Angaben ablesbar wie auf der Blutwaage 11 selber. Dazu sind die Angaben mit einer Kennzeichnung der Blutwaage versehen, von der die Angaben übermittelt wurden.

An der Blutwaage 11 selber ist keine Eingabetastatur dargestellt, wenn auch eine solche in der Praxis vorhanden sein kann. Durch das Fehlen der Eingabetastatur wird gezeigt, dass die Eingabe nicht an der Blutwaage 11, sondern über die Eingabetastatur 25 am Handgerät 23 erfolgt. Die Blutwaage 11 kann deshalb von einer stehenden Person bedient werden. Dazu ist am Handgerät 23 ein Sender 22 (Antenne) und an der Blutwaage ein Empfänger 21 (Antenne) vorhanden. Der Empfänger ist zudem mit der Steuerung der Blutwaage derart verbunden, dass die eingehenden Befehle an diese zur Ausführung übermittelt werden. Es muss daher von der die Blutentnahme überwachenden Person lediglich noch der Blutbeutel aufgelegt und dabei der Schlauch in die Klemme gelegt werden, bzw. der Schlauch aus der Klemme 19 herausgenommen, versiegelt und der Blutbeutel von der Schale 12 weggenommen werden.

Im Handgerät ist ein Strichcode-Scanner 27 integriert. Mit diesem werden z.B. Patientendaten und Blutbeutelnummern erfasst, oder auch die anzusteuernde Blutwaage identifiziert. Dazu ist auf Patientenunterlagen, den Blutbeuteln und auf der Blutwaage jeweils ein Strichcode angebracht.

Figur 2 zeigt eine Blutwaage 11 ohne Eingabetastatur und ohne Anzeige, dafür mit einer Sender/Empfängeranlage 21 für kabellose Übermittlung von Information. Dazu ist ein Computer 31 mit einer Sende/Empfangsanlage 32 und einem Bildschirm 33 wie auch ein Handgerät 21 mit einer Sende/Empfangsanlage für kabellose Übermittlung von Information dargestellt. Die Übermittlung der Information von der Blutwaage 11 zum Computer 31 oder vom Computer 31 zu der Blutwaage 11 kann auch über ein Kabel erfolgen. Es ist jedoch zweckmässig, die kabellose Übermittlung auch hier zu benützen, wenn diese für ein Handgerät 23 ohnehin benötigt wird.

Bei diesem Ausführungsbeispiel werden die Messwerte über Funk an das Handgerät 23 und den Computer 31 übermittelt. Bei beiden kann der übermittelte Messwert angezeigt werden. Wird ein Alarm gegeben, z.B. wenn kein Blut fliesst, so wird der Alarm am Handgerät und am Computer angezeigt und damit sichtbar, hörbar und/oder spürbar gemacht. Gleichzeitig kann eine Angabe der Störungsursache erfolgen. Durch eine Angabe der Störungsursache am Handgerät kann unverzüglich eine Behebung der Störung veranlasst werden, ohne dass eine Anzeige an der Blutwaage zuerst konsultiert zu werden braucht.

Was hier für das Handgerät beschrieben wurde, gilt in gleicher Weise für den Computer 31. Über dessen Tastatur 35 wird der Blutwaage 11 die notwendige Information eingegeben und am Bildschirm werden die Messwerte der Blutwaage angezeigt. Dies erlaubt bei einem Ein-Mann-Betrieb von einem Arbeitsplatz aus mehrere Blutwaagen in einem Blutspenderaum zu überwachen. Die den Computer bedienende Person kann von einem Blutspender die Personaldaten erfassen, die Beutelnummer erfassen und gleichzeitig die für die Blutwaage benötigten Parameter eingeben und an diese übermitteln. Danach kann dieselbe Person die Blutentnahme durchführen, ohne an der Blutwaage Eingaben tätigen zu müssen. Die gemessenen Werte werden von der Blutwaage 11 an den stationären Computer 31 übermittelt und sind somit bereits dem Patienten und Blutbeutel zugeordnet. Ein Drucker kann nun eine Blutbeuteletikette drucken mit den Daten aus dem Computer oder der Blutwaage. Eine solche Lösung ist nicht auf eine kabellose Verbindung angewiesen.

Anstelle des Ausdruckens oder zusätzlich zum Ausdrucken einer Beuteletikette mit alphanumerischen Zeichen, Strichcode oder Punktcode kann auch eine elektronische Etikette mit den entsprechenden Daten bespielt werden. Zum Bespielen der elektronischen Etikette ist am Handgerät 23 ein Schreib/Lesekopf 27 vorgesehen.

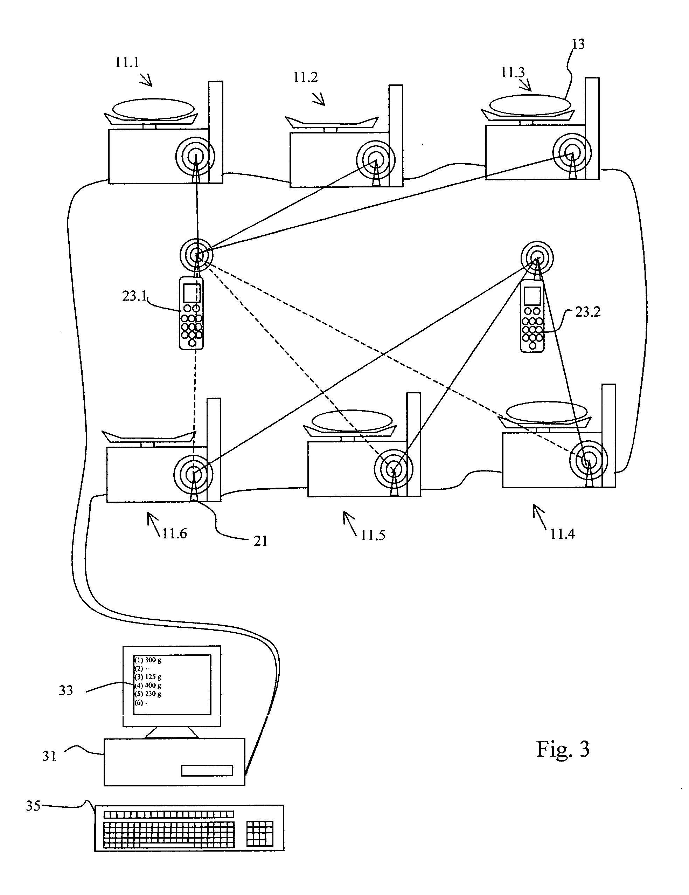

In Figur 3 ist die Überwachungsvorrichtung in einem Blutspenderaum mit sechs Spendeplätzen und zwei Personen zur Durchführung und Überwachung der Blutentnahmen dargestellt. Bei jedem Spendeplatz ist eine Blutwaage 11 (11.1 bis 11.6) mit einem Funk- oder Infrarotsender und -empfänger 21 und einem Kabelanschluss vorhanden. Die Übermittlung zwischen Blutwaage und Computer geschieht in diesem Ausführungsbeispiel über eine andere Verbindung als die Übermittlung zwischen Blutwaagen und Handgeräten. Sie kann auch über eine Speicherkarte erfolgen, die von der Blutwaage bespielt und von einem mit dem Computer verbunden Lesegerät abgelesen werden kann. Vorteilhaft ist jedoch, wenn eine ständige Verbindung aufrechterhalten bleibt und die aktuellen Messwerte aller Blutwaagen 11 auf dem Bildschirm 33 angezeigt werden können.

Den beiden Handgeräten 23.1 und 23.2 sind jeweils drei Blutwaagen 11.1, 11.2 und 11.3, bzw. 11.4, 11.5 und 11.6 zugeordnet (dargestellt durch ausgezogene Geraden zwischen den Antennen). Eines der beiden Handgeräte 23.1 ist derart ausgelegt, dass es sämtliche Alarme empfängt (unterbrochene Linien), während das andere Handgerät 23.2 lediglich die Alarme der ihm zugeordneten Blutwaagen empfängt. Auch andere Meldungen als der Alarm einer Blutwaage können beiden oder allen Überwachungsgeräten übermittelt werden. Es ist auch einstellbar, dass bestimmte Meldungen auf das eine und andere Handgerät 23.1, 23.2 übermittelt werden. Dadurch sind bestimmte Abläufe und Verantwortungen unabhängig vom Spenderplatz den einzelnen Überwachungspersonen zuzuordnen.

Für das Bedienen einer Blutwaage 11 von einem Handgerät 23 aus, wird mit dem Strichcode-Leser 26 im Handgerät 23 ein Strichcode an der zu bedienenden Blutwaage 11 abgelesen, um das Handgerät auf diese Blutwaage einzustellen. Das Einstellen des Handgeräts auf eine spezielle Blutwaage kann auch durch Auswahl über die Tastatur am Handgerät 23 erfolgen. Das Handgerät spricht darauf lediglich diese Blutwaage an. Darauf werden die notwendigen Informationen übermittelt. Es muss daher lediglich der Blutbeutel auf die Blutwaage gelegt werden und der Blutschlauch in die Schlauchklemmvorrichtung eingeführt werden. Danach kann jede Bedienung der Blutwaage 11 am Handgerät 23 und daher stehend oder sitzend geschehen, bis der Blutbeutel 13 wieder von der Blutwaage 11 genommen werden muss. Somit ist es lediglich für die Handhabung des Blutbeutels 13 erforderlich, sich zur Blutwaage 11 hinunter zu bücken.

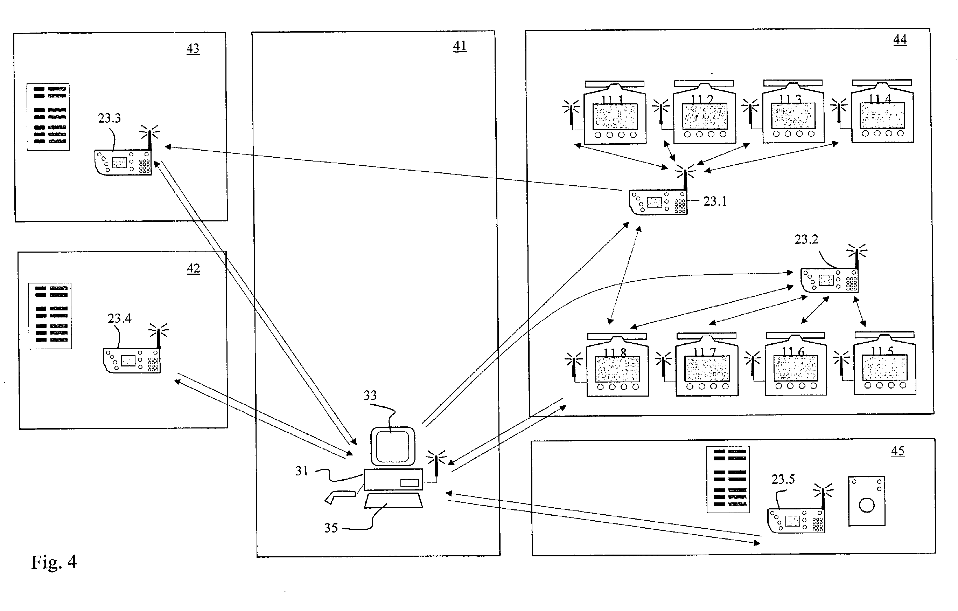

In Figur 4 ist ein ganzes Blutspendezentrum dargestellt. Es sind in einem Blutspendezentrum verschiedene Räume, Zonen oder Ablaufstufen vorgesehen, nämlich z.B.:
- Spenderannahme 41 mit zentraler Überwachung und Datenverwaltung und - verarbeitung und zur Aufnahme von Spenderdaten,
- Untersuchung 42 zur Entnahme von Blutproben und Bestimmung von Blutwerten, Krankheitserregern und anderen Merkmalen des Blutes und des Spenders,
- Arztraum 43 für ärztliche Abklärungen, Abklärung des Gesundheitszustands des Spenders, Information des Spenders und den Notfall,
- Spenderaum 44 für die Blutentnahmen,
- Abschweissen 45 für das Verschweissen und Zwischenlagern der Blutbeutel.

In jedem Raum, ausser der Spenderannahme 41, ist ein Handgerät 23 vorhanden. Dieses wird von einer Person getragen, welche die dem Raum entsprechende Funktion erfüllt. Die Spenderannahme 41 ist mit einem stationären Computer 31 ausgerüstet. Dieser steht in Verbindung mit allen Handgeräten 23. Die Handgeräte ihrerseits stehen in Verbindung miteinander und sind in der Lage, die Blutwaagen 11 zu bedienen.

Im Untersuchungsraum 42 werden Blutwerte wie Temperatur und Hämoglobin ermittelt. Diese werden ins Handgerät eingegeben und direkt an die Datenbank im zentralen Computer 31 weitergeleitet. Damit die Zuordnung der Blutwerte zur richtigen Probe und zum richtigen Spender erfolgt, werden mit dem Handgerät jeweils Strichcodes abgelesen, die der Probe und dem Spender zugeordnet sind. Mit dem Handgerät kann auch auf die Datenbank zugegriffen werden. So werden z.B. von der Datenbank dem Spender zugeordnete, früher erfasste Daten, an das Handgerät übermittelt. Auf diese Weise kann z.B. ein Verlauf der Hämoglobinwerte überwacht oder auf früher festgestellte Krankheitserreger aufmerksam gemacht werden.

Im Arztraum 43 kann der Arzt mit dem Handgerät die Datenbank abfragen und hat so nach Einlesen des Strichcodes des Spenders die gesamte Spendergeschichte zur Hand, ohne eine separate Kartei führen zu müssen. Er kann diese Datei aktualisieren mit seinen ärztlichen Untersuchungsergebnissen. Er kann direkt eine Spendernummer sperren lassen, wenn er feststellt, dass ein Spender nicht spenden darf. Damit ist vorgebeugt, dass diesem Spender irrtümlich Blut abgenommen wird. Bei nachträglicher Sperrung des Spenders ermittelt der zentrale Computer den gesperrten Blutbeutel, der dann beim Abschweissen 45 eliminert wird. Das Eruieren des gesperrten Blutbeutels erfolgt in der Folge automatisch beim Einlesen der Strichcodes des gesperrten Beutels, was bei jeder Handlung mit dem Blutbeutel erfolgt, z.B. beim Verschweissen, beim Einlagern im Kühlraum und spätestens beim Umladen für den Transport.

Der Arzt ist über sein Handgerät 23.3 immer erreichbar von jedem andern Handgerät aus. So kann er nach Notwendigkeit, Bedürfnis oder Wunsch herbeigerufen werden.

Die Handhabung der Waagen 11.1 bis 11.8 im Spenderaum erfolgt wie im vorherigen Beispiel über die Handgeräte 23.1 und 23.2. Wird z.B. der Arzt herbeigerufen zur Verstärkung des Spenderaum-Teams, so kann auch er jede Waage über sein Handgerät 23.3 bedienen. Die Übertragung von Datensätze vom zentralen Computer 31 auf eine Waage, z.B. die Blutwaage 11.8, erfolgt über dieselbe Sendeanlage, wie die Kommunikation vom Computer 31 zu den Handgeräten 23. Beim Abschweissen der Blutbeutel und bei der Lagerhaltung wird die gelungene Verschweissung mit dem Handgerät 23.5 elektronisch dem zentralen Computer 31 gemeldet. Die Lagerverwaltung erfolgt ebenfalls durch Einlesen des Strichcodes des Blutbeutels beim Einlagern sowie beim Auslagern der Blutbeutel.

Die Funktionen des Handgerätes können durch Ablesen eines Strichcodes an einem Gerät eingestellt werden. So liest eine Person mit dem Handgerät 23.1 den Strichcode der Waage 11.1 ab, um die Waage 11.1 zu bedienen. Wird anschliessend der Strichcode eines Blutbeutels abgelesen, so wird dieser Blutbeutel der abgelesenen Waage zugeordnet. Um die gelagerten Blutbeutel, die abgeholt werden von eine verarbeitenden Betrieb zu erfassen, liesst man mit dem Lesegerät des Handgerätes 23.5 den Strichcode des Lagerfaches ab. Damit weiss der Computer, ob die nachfolgend abgelesene Beutelnummer eingelagert wird, oder ausgelagert wird. Denn im Computer sind die eingelagerten Blutbeutelnummern bereits den Lagerfächern zugeordnet notiert, bzw. die noch nicht eingelagerten Blutbeutel sind als in Bearbeitung erfasst. Werden die im Fach gelagerten Blutbeutel zum Abtransport bereitgemacht und in einen Behälter gelegt, kann der Computer 31 einen Lieferschein generieren, der den Behälterinhalt spezifiziert. Die Daten über die Blutbeutel können zudem dem Behälter zugeordnet elektronisch an den das Spenderblut weiterverabeitenden Betrieb übermittelt werden.

Zum Erfassen von Arbeitsschritten oder Leistungen liegen in den verschiedenen Räumen Listen 51 vor. Die Listen weisen für jeden Arbeitsschritt und jede Leistung einen Strichcode auf. Anstelle eines Strichcodes kann auch ein, Punktcode, Transponder oder ein anderer berührungslos ablesbarer Informationsträger vorliegen. Diese Informationsträger sind jedenfalls mit dem Handgerät ablesbar. Die Informationsträger enthalten die Eingabeinformation. Auf der Liste ist neben dem elektronisch lesbaren Informationsträger jeweils die entsprechende Leistung, der Arbeitsschritt oder der Bedienungsbefehl mit alphanumerischen Zeichen lesbar angegeben.

Diese Vernetzung von lesefähigen Handgeräten 23 mit einem zentralen Computer 31 und einer zentralen Datenbank vereinfacht die Überwachung von Blutspenden von der Erfassung von Spenderdaten über die Untersuchungen, die Bedienung der Blutwaagen 11 bis hin zum Blut verarbeitenden Betrieb. Dabei wird die Sicherheit durch wiederholte automatisierte Kontrollen wesentlich erhöht. Die Datenbank im zentralen Computer ist jederzeit aktuell nachgeführt, dank dem die mit den Handgeräten erfassten und generierten Daten unmittelbar nach deren Erfassung oder Generierung an die Datenbank übermittelt werden.

Sind die Datenträger nicht Strichcodes oder Punktcodes, sondern elektronische Datenträger, wie z.B. mit einem Transponder oder "Bluetooth"-Chips verbundene Speicher, so können Informationen bei jedem Arbeitsschritt auf diese Speicher geschrieben werden. Dadurch kann das Erstellen eines Lieferscheines mit den erfassten Daten entfallen. Jeder Blutbeutel trägt dann schon die spezifischen Daten in seiner elektronischen Etikette bei sich. Auf eine solche Etikette können weiter Lagerdaten wie Lagerzeiten und Lagertemperaturen etc. gespeichert werden. Dazu kann ein Lagerplatz mit einem Sensor und/oder einer Uhr versehen sein, welche mit einem Schreibgerät verbunden sind. Es kann auch jede elektronische Etikette einen Sensor aufweisen, der Zeit und/oder Temperatur misst und die gemessenen Daten in den Speicher schreibt. So kann beispielsweise in regelmässigen Abständen die Temperatur oder /und der Zeitpunkt einer Temperaturveränderung festgehalten werden.

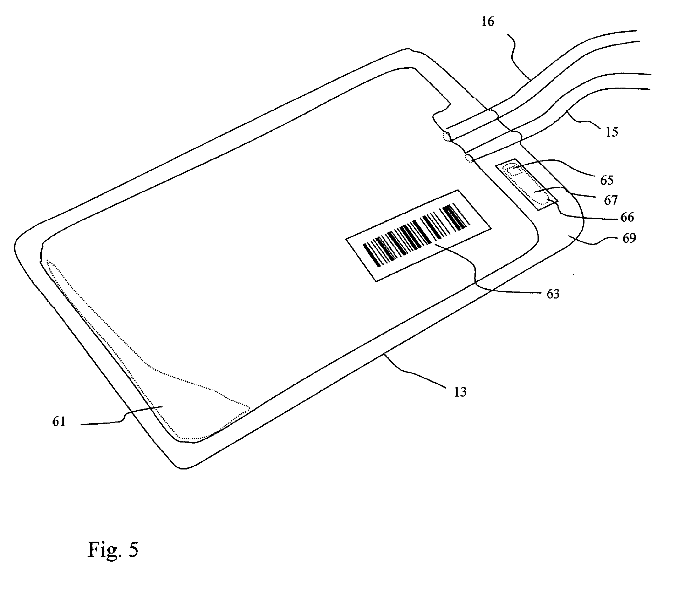

In Figur 5 ist ein Blutbeutel 13 dargestellt. Der Blutbeutel hat wie üblich zwei Schläuche 15, 16 zum Befüllen des Blutbeutels und für die Transfusion des Blutes zu einem Blutempfänger. Die Schläuche 15, 16 sind im Rand des Blutbeutels eingeschweisst. Der Blutbeutel 13 enthält ein Antikoagulansmittel 61. Auf dem Blutbeutel ist eine Strich-Code-Etikette 63 aufgeklebt. Der Strichcode enthält unveränderliche Daten, die zumindest den Beutel identifizieren. Mit Hilfe der elektronischen Datenbank lassen sich aufgrund der Identifikation dem Blutbeutel 13 sämtliche erfassten Daten zuordnen. Alternativ oder zusätzlich ist der Blutbeutel mit einem elektronischen Speicher 65 versehen, der über eine Transponderschlaufe 66 oder einen "Bluetooth"-Chip mit einem Schreib/Lesekopf beschreibbar und auslesbar ist. In diesem Speicher können sämtliche erfassten Daten gespeichert sein. Es können auch lediglich während Transport und Lagerung entstehende Daten darauf gespeichert werden.

Die elektronische Etikette 67 mit dem Speicher 65 kann mit der Strichcode-Etikette oder separat davon auf den Blutbeutel aufgeklebt werden. In der Figur 5 ist die elektronische Etikette 67 in den Rand 69 des Blutbeutels 13 eingeschweisst. Damit ist sie untrennbar vom Blutbeutel 13 und unverlierbar. Zudem ist der Chip und die Transponderschlaufe durch die Steifigkeit des Beutelrandes geschützt. Der Beutelrand kann zudem zusätzliche Versteifungen aufweisen.

## Patentansprüche

1. Vorrichtung zur Überwachung von Blutentnahmen, umfassend:
- wenigstens eine Blutwaage (11)
- mit einer Beutelschale (12) zum Auflegen eines Blutbeutels (13) während einer Blutentnahme und
- mit einer Wägeeinrichtung zum Messen des Beutelgewichts, und
- mit einer Empfangseinrichtung (21)
- und wenigstens ein von der Blutwaage getrenntes Eingabegerät,
- mit einem Eingabefeld und
- mit einer Absendeeinrichtung
- mittels welcher Absendeeinrichtung und Empfangseinrichtung das Eingabegerät mit der Blutwaage elektronisch kommunizieren kann, **dadurch gekennzeichnet,**
- **dass** das Eingabegerät
- zur Eingabe und Übermittlung von Bedienungsbefehlen zur Bedienung der Blutwaage ausgelegt ist und
- **dass** die Blutwaage
- einen Steuerkreislauf zum Ausführen der mit der Empfangseinrichtung empfangenen Bedienungsbefehle aufweist.

2. Vorrichtung zur Überwachung einer Blutentnahme gemäss Anspruch 1, bei der
- die Blutwaage (11) ausgerüstet ist
- mit einer Absendeeinrichtung (21) zum Senden eines Messwertes,
- und das von der Blutwaage (11) getrennte Eingabegerät ein Überwachungsgerät (23,31) ist, das ausgerüstet ist
- mit einer Empfangseinrichtung (22) zum Empfangen eines Messwertes einer Blutwaage
- und mit einer Anzeige (24,33) zum Anzeigen des Messwertes.

3. Vorrichtung nach einem der vorangehenden Ansprüche, **dadurch gekennzeichnet, dass** die Absendeeinrichtung und die Empfangseinrichtung kabellos verbunden sind, so dass die Übertragung von Bedienungsbefehlen und Messwerten kabellos erfolgt.

4. Vorrichtung nach Anspruch 3, **dadurch gekennzeichnet, dass** das Eingabe/Überwachungsgerät ein mobiles Handgerät (23) ist, das von einer eine Blutentnahme überwachenden Person mitgeführt werden kann, und dessen Abmessungen insbesondere so klein sind, dass es in eine Kitteltasche passt.

5. Vorrichtung nach Anspruch 4, **dadurch gekennzeichnet, dass** mehrere Blutwaagen (11) und mehrere Eingabe/Überwachungsgeräte (31,23) vorhanden sind, wobei wenigstens ein mobiles Handgerät (23) kabellos mit einer Blutwaage (11) verbunden ist.

6. Vorrichtung nach Anspruch 5, **dadurch gekennzeichnet, dass** eine Zuordnung einer Blutwaage (11) zu dem mobilen Handgerät (23) oder den mobilen Handgeräten programmierbar ist.

7. Vorrichtung nach einem der Ansprüche 5 oder 6, **dadurch gekennzeichnet, dass** die Handgeräte (23) derart ausgelegt sind, dass sie Informationen an Handgeräte (23) senden und von Handgeräten (23) empfangen können.

8. Vorrichtung nach einem der vorangehenden Ansprüche, **dadurch gekennzeichnet, dass** ein Lesekopf (27) am Eingabe/Überwachungsgerät (31,23) angeschlossen ist, insbesondere der Lesekopf (27) im Handgerät (23) integriert ist.

9. Vorrichtung nach einem der vorangehenden Ansprüche, **dadurch gekennzeichnet, dass** die Blutwaage ein elektronisches Alarmsignal aussenden kann und im Eingabe/Überwachungsgerät (31,23) ein Alarmgeber integriert ist, der ein akustisches, visuelles und/oder taktiles Signal gibt, wenn eine Blutwaage (11) ein Alarmsignal sendet.

10. System zur Überwachung von Blutentnahmen mit einer Blutwaage und einem von der Blutwaage getrennten Eingabe- oder Überwachungsgerät gemäss einem der Ansprüche 1 bis 9, und einem Schreib/Lesekopf am Eingabe/Überwachungsgerät, sowie einem mit einem Blutbeutel verbundenen elektronischen Datenträger, wobei mit dem Eingabe/Überwachungsgerät über den Schreib/Lesekopf medizinische Daten auf den Datenträger geschrieben und vom Datenträger ausgelesen werden können.

11. System gemäss Anspruch 10, **gekennzeichnet durch** einen mit dem Datenträger gekoppelten Sensor zur Erfassung von Lagerdaten des Blutbeutels.

12. System gemäss einem der Ansprüche 10 oder 11, **gekennzeichnet durch** mit Schreibköpfen verbundene Sensoren an wenigstens einer Lagerstelle für Blutbeutel.

13. Verfahren zur Überwachung von Blutentnahmen, **dadurch gekennzeichnet, dass** einer Blutwaage von einem von der Blutwaage getrennten Eingabe/Überwachungsgerät aus Bedienungsbefehle übermittelt werden.

14. Verfahren nach Anspruch 13, **dadurch gekennzeichnet, dass** von der Blutwaage an das Überwachungsgerät Messwerte übermittelt und am Überwachungsgerät angezeigt werden.

15. Verfahren nach Anspruch 13 oder 14, **dadurch gekennzeichnet, dass** von der Blutwaage an das Eingabe/Überwachungsgerät ein elektronisches Alarmsignal übermittelt und das Eingabe/Überwachungsgerät als Reaktion auf das eintreffende Alarmsignal ein akustisches, visuelles und/oder taktiles Signal aussendet.
